# FASCICULE DE BREVET EUROPEEN

(11) **EP 2 007 869 B1**
(45) Date de publication et mention de la délivrance du brevet: **01.04.2015**
(21) Numéro de dépôt: 06764568.9
(22) Date de dépôt: 20.04.2006
(51) Int. Cl.: C12M 1/00, C12M 3/02

(54) **RÉCIPIENT DESTINÉ À LA CULTURE DE CELLULES COMPRENANT DES CHAMBRES DE CONFINEMENT ET DE NUTRITION**
FÜR DIE ZELLKULTUR VORGESEHENES BEHÄLTNIS MIT ARREST- UND NAHRUNGSKAMMERN
RECEPTACLE DESIGNED FOR CELL CULTURE INCLUDING CONFINEMENT AND NUTRITION CHAMBERS

(43) Date de publication de la demande: 31.12.2008
(73) Titulaire: Maco Pharma S.A., 59420 Mouvaux (FR)
(72) Inventeur: HERON, Antoine, F-59250 Halluin (FR)
(74) Mandataire: Herrou, Nathalie
(86) Numéro de dépôt international: PCT/FR2006/000867
(87) Numéro de publication internationale: WO 2007/122299

(56) Documents cités:
- US-A- 5 507 133
- US-A- 6 127 168
- US-B1- 6 297 046

## Description

L'invention concerne un récipient destiné à la culture de cellules ainsi qu'un procédé de mise en culture des cellules avec un tel récipient.

Le récipient selon l'invention est destiné à la culture de cellules en suspension et notamment à la culture simultanée de différents types de cellules.

L'avènement des cultures in vitro de cellules directement transplantables chez l'homme est à l'origine du développement de différents types de récipients pour le conditionnement desdites cultures. Utilisés dans un cadre médical pour la préparation de produits de thérapie cellulaire et génique, ces récipients doivent présenter dés garanties en matière de confinement des cellules, de prétention des risques de contamination et des erreurs techniques de manipulation. Lesdits récipients se doivent donc de respecter les règles strictes de bonne pratique des produits transfusables pour le conditionnement clos et le transfert des cellules.

De fait, l'utilisation de poches souples s'est accrue pour cultiver des cellules exploitées en thérapeutique humaine, notamment dans le cadre du développement des protocoles cliniques d'expansion ex *vivo* des cellules souches hématopoïétiques issues de prélèvement de moelle osseuse, de sang périphérique et de sang de cordon. Le document US 6 297 046 illustre de telles poches souples destinées à la culture de cellules.

Cependant, certains protocoles cliniques d'expansion nécessitent de cultiver simultanément plusieurs types de cellules. Or, les pochés souples, posées à plat dans l'incubateur lors de la phase de culture, ne sont pas adaptées pour ce type de culture. En effet, la co-culture nécessite de concentrer les cellules dans un espace confiné afin de favoriser leur communication.

La co-culture est en général réalisée dans des puits à fond rond. Cette culture en système ouvert n'est pas compatible avec un usage clinique en routine.

Il convient de développer un récipient qui puisse respecter les bonnes pratiques, notamment en terme de confinement et de risque de contamination.

A cet effet et selon un premier aspect, l'invention propose un récipient destiné à la culture de cellules, comprenant une première et une deuxième feuilles en matériau thermoplastique souple solidarisées l'une à l'autre au voisinage de leur périphérie de sorte à former un volume intérieur, le récipient comprenant au moins une première voie d'accès au volume intérieur qui est située sur un bord transversal du récipient et agencée pour permettre l'introduction et/ou la récupération des cellules. Le récipient comporte une ligne de pliure longitudinale selon laquelle le récipient peut être plié de sorte à former, respectivement de part et d'autre de ladite ligne de pliure, un premier et un deuxième compartiments dans le volume intérieur. Le premier compartiment est pourvu d'un premier déflecteur longitudinal qui est agencé pour former, entre un bord longitudinal du récipient et ledit premier déflecteur, une première chambre de confinement et, entre ledit premier déflecteur et la ligne de pliure, une première chambre de nutrition, lesdites premières chambres de confinement et de nutrition étant en communication fluidique. La première voie d'accès débouche dans la chambre de nutrition. Le deuxième compartiment est pourvu d'un deuxième déflecteur longitudinal qui est agencé pour former, entre l'autre bord longitudinal du récipient et ledit deuxième déflecteur, une deuxième chambre de confinement et, entre ledit deuxième déflecteur et la ligne de pliure, une deuxième chambre de nutrition, lesdites deuxièmes chambres de confinement et de nutrition étant en communication fluidique.

Selon un deuxième aspect, l'invention propose un procédé de mise en culture de cellules en suspension, comprenant les étapes:
- introduire des cellules en suspension dans un récipient selon le premier aspect de l'invention par la première voie d'accès,
- plier ledit récipient le long de la ligne de pliure,
- incliner le récipient plié dans le plan vertical jusqu'à amener les cellules en suspension dans les chambres de confinement.

D'autres objets et avantages de l'invention apparaîtront au cours de la description qui suit en référence aux dessins annexés dans lesquels :
- La figure 1 représente de façon schématique une vue de face du récipient selon un mode de réalisation de invention;
- la figure 2 représente de façon schématique une vue en perspective du récipient de la figure 1 contenant des cellules, lorsqu'il est plié,
- les figures 3 à 5 représentent de façon schématique une coupe transversale du récipient de la figure 1 maintenu plié par un assemblage à rainure et languette, sur un crochet ou sur un support, respectivement.
- la figure 6 représente de façon schématique une vue en perspective d'un récipient selon un autre mode de réalisation de l'invention contenant les cellules et le milieu de culture et lorsqu'il est plié,
- la figure 7 représente de façon schématique une vue de face du récipient selon un autre mode de réalisation,
- les figures 8a à 8c représentent de façon schématique le récipient de la figure 1 à différentes étapes de mise en culture des cellules,
- la figure 9 représente de façon schématique le récipient de la figure 1 en position pliée avec une pince.

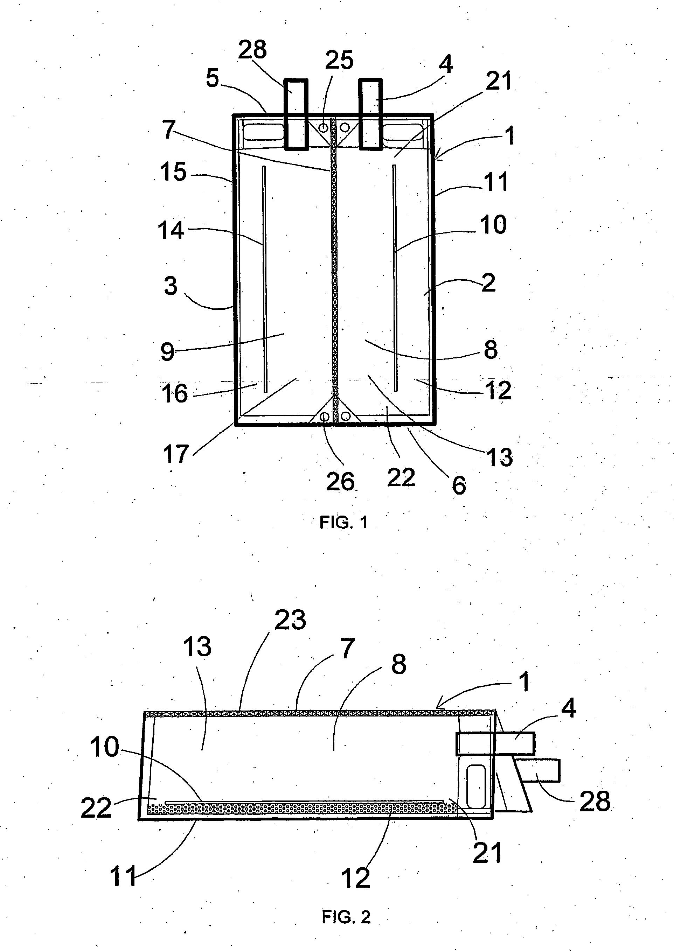

Les termes "supérieur" et "inférieur" sont définis par rapport à la position du récipient lorsque la première voie d'accès est le plus haut, comme représenté sur la figure 1, c'est-à-dire la position du récipient au moment de l'introduction des cellules. Le plan du récipient définit également le plan "vertical".

L'invention propose un récipient destiné à la culture de cellules, notamment les cellules non adhérentes. En particulier, le récipient permet la culture de cellules provenant d'une ou plusieurs populations dans un espace confiné.

Par exemple, le récipient est utilisé pour l'activation des lymphocytes infiltrant la tumeur (TIL ou Tumor Infiltrating Lymphocytes) par coculture avec une autre population de cellules.

En référence à la figure 1, le récipient 1 comprend une première et une deuxième feuilles 2,3 en matériau thermoplastique souple solidarisées l'une à l'autre au voisinage de leur périphérie de sorte à former un volume intérieur.

Des exemples de matériau thermoplastique souple sont le polychlorure de vinyle, l'alcool polyvinylique, le polyéthylène téréphtalate ou le polystyrène. D'autres exemples sont l'EVA, des polyoléfines tel que le polypropylène, le polyéthylène et/ou le polybutadiène, ou des polymères fluorés tel que l'éthylène-propylène fluoré (FEP), le Teflon® PFA ou le polytrétrafluoréthylène (PTFE). Ces derniers exemples de matériau présentent l'avantage d'être perméables aux gaz et stérilisables.

Selon une réalisation possible, les feuilles sont soudées. Toutefois, les feuilles peuvent également être solidarisées par une méthode différente, notamment par collage.

Le récipient 1 comprend au moins une première voie d'accès 4 au volume intérieur qui est située sur un bord transversal du récipient et agencée pour permettre l'introduction et/ou la récupération des cellules.

Le bord transversal sur lequel est située la voie d'accès est par définition le bord supérieur 5, l'autre bord étant le bord inférieur 6.

Selon une réalisation, la première voie d'accès 4 est formée d'une partie de tubulure, insérée entre les deux feuilles.

Selon l'invention, le récipient comporte une ligne de pliure longitudinale 7 selon laquelle le récipient peut être plié de sorte à former, respectivement de part et d'autre de ladite ligne de pliure, un premier et un deuxième compartiments 8,9 dans le volume intérieur.

Le premier compartiment est pourvu d'un premier déflecteur longitudinal 10 qui est agencé pour former, entre un bord longitudinal 11 du récipient et ledit premier déflecteur 10, une première chambre de confinement 12 et, entre ledit premier déflecteur 10 et la ligne de pliure 7, une première chambre de nutrition 13, lesdites premières chambres de confinement 12 et de nutrition 13 étant en communication fluidique.

Le deuxième compartiment 9 est pourvu d'un deuxième déflecteur 14 longitudinal qui est agencé pour former, entre l'autre bord longitudinal 15 du récipient et ledit deuxième déflecteur 14, une deuxième chambre de confinement 16 et, entre ledit deuxième déflecteur 14 et la ligne de pliure 7, une deuxième chambre de nutrition 17, lesdites deuxièmes chambres de confinement 16 et de nutrition 17 étant en communication fluidique.

Comme le montrent les différentes figures, la première voie 4 d'accès débouche dans la chambre de nutrition 13.

Chacune des chambres de confinement 12,16 est destinée à recevoir des cellules à cultiver, notamment des cellules non adhérentes en suspension dans un peu de milieu de culture. Les chambres de confinement 12,16 définissent un espace présentant un volume réduit, ce qui permet de maintenir une forte densité cellulaire.

Le volume des chambres de confinement 12,16 est déterminé par la taille des déflecteurs 10,14.

Lors de la phase de culture, le récipient 1 est plié selon la ligne de pliure 7 et positionné horizontalement dans le plan vertical comme illustré sur la figure 2, de sorte que les cellules en suspension dans les chambres de confinement 12,16 sédimentent par gravité au niveau des arêtes 18,19 définies par la jonction des deux feuilles 2,3 formant le récipient 1.

Le fait de plier le récipient permet d'exploiter les deux arrêtes 18,19 longitudinales du récipient.

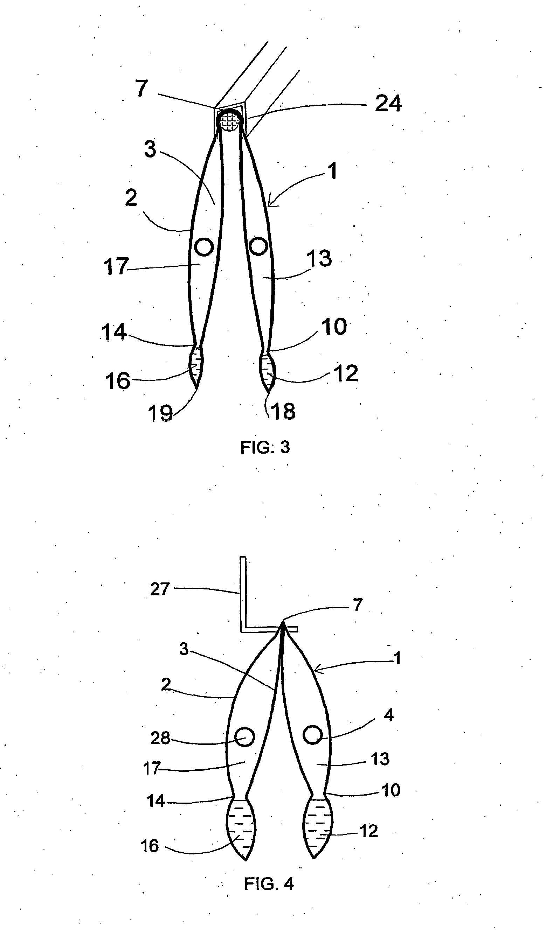

Dans cette réalisation et comme le montrent les figures 3 à 5, les fonds des chambres de confinement 12,16 présentent une forme en V, favorisant la concentration des cellules en culture.

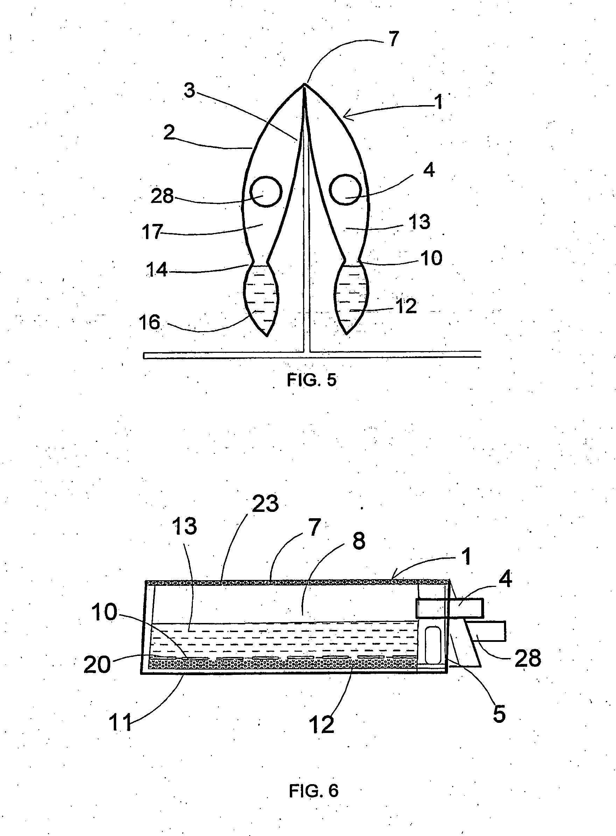

Chacune des chambres de nutrition 13,17 est destinée à recevoir un milieu de culture servant à alimenter les cellules se trouvant dans chacune des chambres de confinement 12,16 (figure 6).

Un premier et un deuxième déflecteur 10,14 séparent respectivement la première chambre de confinement 12 de la première chambre de nutrition 13 et la deuxième chambre de confinement 16 de la deuxième chambre de nutrition 17.

Le déflecteur 10 ou 14 contraint les cellules en culture à rester dans la chambre de confinement 12 ou 16. De plus, lors de l'ajout ou le retrait de milieu de culture, le déflecteur 10 ou 14 empêche les cellules d'être déplacées ou remises en suspension.

Par exemple, au moins un déflecteur 10 ou 14 est formé d'un cordon de soudure 20 entre les feuilles, ledit cordon s'étendant longitudinalement à l'intérieur du compartiment en formant au moins une zone de communication transversale entre les chambres de confinement 12,16 et de nutrition 13,17.

Selon la réalisation de la figure 1, le cordon est distant des bords du récipient de sorte à former deux zones de communication transversale 21,22, respectivement supérieure et inférieure.

Dans cette réalisation, les cellules en suspension sont isolées dans les chambres de confinement 12,16 et l'échange avec le milieu de culture s'effectue au niveau des extrémités des déflecteurs 10,14.

Selon une variante représentée sur la figure 6, le cordon de soudure 20 est discontinu. Le cordon de soudure est notamment formé d'une succession de points de soudure de faible longueur régulièrement espacés.

Les espaces entre les points de soudure augmentent les échanges fluidiques entre la chambre de confinement 12 ou 16 et le milieu de culture placé dans la chambre de nutrition 13 ou 17.

De façon avantageuse, les points de soudure adjacents aux bords du récipient sont accolés aux bords du récipient. Ainsi, lorsque du milieu de culture est ajouté dans le récipient disposé horizontalement, les points de soudure empêchent le milieu de culture de s'écouler directement sur les cellules en culture et donc de les remettre ensuspension.

Dans cette réalisation, la voie d'accès 4 est avantageusement placée juste à côté du déflecteur 10, de sorte que le milieu de culture s'écoule sur le point de soudure accolé au bord 5 du récipient.

En relation avec la figure 2, le, récipient 1 est plié selon la ligne de pliure 7. Par exemple, la ligne de pliure est sous forme d'un pli réalisé dans le récipient, notamment rigidifié par exemple par soudage.

Selon la figure 1, la ligne de pliure 7 comprend au moins un cordon de soudure 23 entre les feuilles.

En variante non représentée et afin de faciliter le pliage du récipient, la ligne de pliure 7 comprend deux cordons de soudures disposés l'un à côté de l'autre.

Lors de la phase de culture, le récipient doit être maintenu en position pliée. Afin de maintenir le récipient cette position pliée, il est possible d'utiliser par exemple une pince ou un assemblage à rainure et languette 24 (cf. figure 3).

Selon une autre réalisation représentée sur la figure 1, le récipient est muni de deux paires d'ouvertures d'accrochage 25,26, par exemple sous forme d'oeillets. Chaque paires d'ouverture est située respectivement à proximité des bords transversaux supérieur et inférieur 5,6 du récipient. Pour chaque paire d'ouverture d'accrochage, les ouvertures sont situées de part et d'autre de la ligne de pliure 7.

Pour la culture des cellules, le récipient plié selon la ligne de pliure 7 est suspendu à des crochets 27 par l'intermédiaire desdites ouvertures d'accrochage 25,26, comme le montre la figure 4.

En variante représenté sur la figure 5, le récipient plié repose au niveau de la ligne de pliure 7 sur un support.

Selon une réalisation représentée sur la figure 1, la ligne de pliure 7 s'étend du bord supérieur 5 au bord inférieur 6 du récipient, de sorte à former deux compartiments 8,9 isolés l'un de l'autre.

Le récipient comprend alors une deuxième voie d'accès 28 débouchant dans la deuxième chambre de nutrition 17, ladite deuxième voie 28 étant prévue sur un bord transversal du récipient.

Notamment, les première et deuxième voies d'accès 4,28 sont situées sur le bord supérieur 5 du récipient, respectivement de part et d'autre de la ligne de pliure 7.

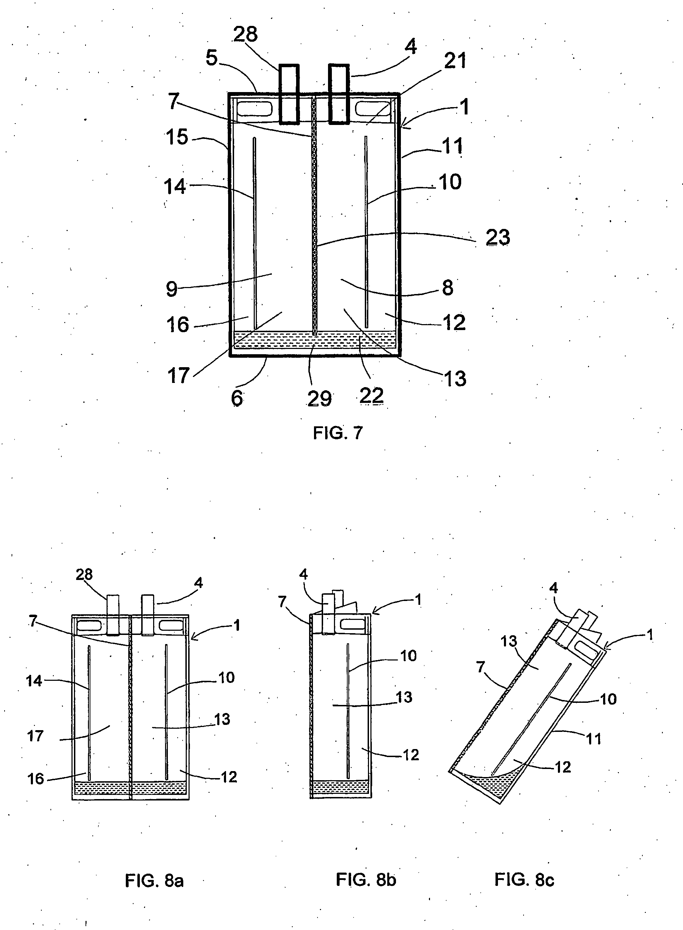

Selon une variante représentée sur la figure 7, la ligne de pliure 7 s'étend du bord supérieur 5 jusqu'à proximité du bord inférieur 6, de sorte à former une zone inférieure de communication fluidique 29 entre les compartiments 8,9.

Cette réalisation permet un ensemencement plus facile et rapide des cellules, puisqu'une seule voie d'accès est utilisée pour l'introduction des cellules. De plus, cette variante apporte également l'avantage dé pouvoir répartir de façon homogène la quantité de cellules dans les deux compartiments 8,9.

En variante non représentée, le récipient 1 comprend une troisième et/ou quatrième voie d'accès débouchant dans la première et/ou deuxième chambre de confinement 12,16, respectivement. Ces troisième et quatrième voie d'accès sont situées par exemple sur le bord supérieur 5 du récipient.

Ces troisième et quatrième voie d'accès permettent le retrait des cellules du récipient de façon aisée.

On décrit maintenant une utilisation particulière du récipient.

Le récipient est destiné à la culture de cellules, notamment des cellules non adhérentes issus de différentes populations.

Ce récipient est particulièrement adapté à l'activation des TIL (Tumor infiltrating lymphocytes) avec une autre population de cellules.

Il est possible d'intégrer le récipient selon l'invention dans un système clos.

Le système comprend alors au moins deux éléments reliés entre eux en circuit clos au moyen d'une tubulure, l'un au moins desdits éléments étant un récipient selon l'invention, la tubulure étant connectée par une première extrémité à une voie d'accès du récipient et par une deuxième extrémité à un autre élément du système, de sorte à permettre le passage des cellules et/ou des fluides entre les éléments du système.

En particulier, le récipient est relié à une première et deuxième poche de culture.

Selon un deuxième aspect de l'invention, on décrit maintenant un procédé de mise en culture de cellules en suspension, comprenant les étapes:
- introduire des cellules en suspension dans un récipient 1 selon le premier aspect de l'invention par la première voie d'accès 4,
- plier ledit récipient le long de la ligne de pliure 7,
- incliner le récipient plié dans le plan vertical jusqu'à amener les cellules en suspension dans les chambres de confinement 12,16.

En relation avec les figures 8a à 8c, le récipient 1 étant initialement vide, la première étape consiste en l'ensemencement des cellules dans le récipient, c'est-à-dire l'introduction des cellules en suspension dans un minimum de milieu de culture via la première et/ou la deuxième voie d'accès 4,28.

Pendant cette étape illustrée sur la figure 8a, le récipient 1 est tenu verticalement et les cellules se retrouvent le long du bord transversal inférieur 6 du récipient.

Comme représenté sur la figure 8b, le récipient est ensuite plié selon la ligne de pliure 7 pour former les deux compartiments 8,9.

Selon la figure 8c, on incline ensuite doucement le récipient de 90° dans le plan vertical de sorte à amener la ligne de pliure 7 en position horizontale et les cellules en suspension dans les chambres de confinement 12,16.

A la fin de cette étape, les cellules sont concentrées dans le fond des chambres de confinement 12,16, le long des bords longitudinaux 11,15 du récipient.

L'étape suivante d'introduction du milieu de culture est réalisée lorsque le récipient est plié et disposé horizontalement dans le plan vertical. Le milieu de culture est introduit dans récipient via la première et la deuxième voie d'accès 4,28.

De façon similaire, lorsqu'il est nécessaire de renouveler ou retirer le milieu de culture, une partie ou tout le milieu de culture est retiré du récipient via la première et la deuxième voie d'accès 4,28 lorsqu'il est dans la même position que celle décrite pour l'introduction du milieu de culture.

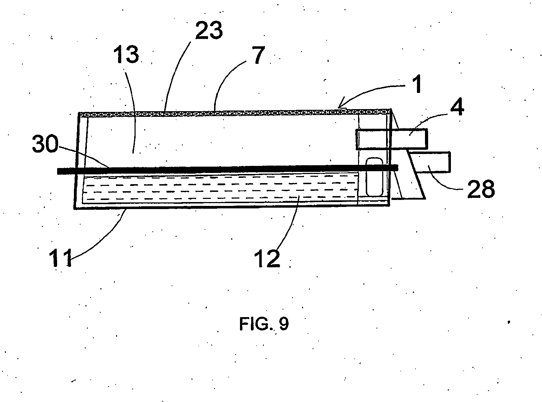

Comme représenté sur la figure 9, lors des étapes d'introduction ou de retrait du milieu, on peut utiliser une pince ou un clamp 30 de longueur égale ou supérieure à la longueur du récipient afin d'isoler complètement les chambres de confinement 12,16 des chambres de nutrition 13,17. Ainsi, les cellules ne sont pas perturbées pendant l'ajout, le renouvellement ou le retrait du milieu de culture.

## Revendications

1. Récipient (1) destiné à la culture de cellules, comprenant une première et une deuxième feuilles (2,3) en matériau thermoplastique souple solidarisées l'une à l'autre au voisinage de leur périphérie de sorte à former un volume intérieur, le récipient comprenant au moins une première voie d'accès (4) au volume intérieur qui est située sur un bord transversal du récipient et agencée pour permettre l'introduction et/ou la récupération des cellules, **caractérisé en ce que** :
- le récipient comporte une ligne de pliure longitudinale (7) selon laquelle le récipient peut être plié de sorte à former, respectivement de part et d'autre de ladite ligne de pliure (7), un premier et un deuxième compartiments (8,9) dans le volume intérieur,
- le premier compartiment (8) est pourvu d'un premier déflecteur longitudinal (10) qui est agencé pour former, entre un bord longitudinal (11) du récipient et ledit premier déflecteur (10), une première chambre de confinement (12) et, entre ledit premier déflecteur (10) et la ligne de pliure (7), une première chambre de nutrition (13), lesdites premières chambres de confinement et de nutrition (12,13) étant en communication fluidique,
- la première voie d'accès (4) débouche dans la chambre de nutrition (12), et
- le deuxième compartiment (9) est pourvu d'un deuxième déflecteur longitudinal (14) qui est agencé pour former, entre l'autre bord longitudinal (15) du récipient et ledit deuxième déflecteur (14), une deuxième chambre de confinement (16) et, entre ledit deuxième déflecteur (14) et la ligne de pliure (7), une deuxième chambre de nutrition (17), lesdites deuxièmes chambres de confinement et de nutrition (16,17) étant en communication fluidique.

2. Récipient selon la revendication 1, **caractérisé en ce qu'**au moins un déflecteur est formé d'un cordon de soudure (20) entre les feuilles, ledit cordon s'étendant longitudinalement à l'intérieur du compartiment en formant au moins une zone de communication transversale entre les chambres de confinement et de nutrition.

3. Récipient selon la revendication 2, **caractérisé en ce que** le cordon est distant des bords du récipient de sorte à former deux zones de communication transversale (21,22), respectivement supérieure et inférieure.

4. Récipient selon la revendication 2 ou 3, **caractérisé en ce que** le cordon de soudure (20) est discontinu.

5. Récipient selon l'une quelconque des revendications 1 à 4, **caractérisé en ce que** la ligne de pliure (7) comprend au moins un cordon de soudure (23) entre les feuilles.

6. Récipient selon la revendication 5 **caractérisé en ce que** la ligne de pliure (7) comprend deux cordons de soudures disposés l'un à côté de l'autre.

7. Récipient selon l'une quelconque des revendications 1 à 6, **caractérisé en ce que** la ligne de pliure (7) s'étend du bord supérieur (5) au bord inférieur (6) du récipient, de sorte à former deux compartiments (8,9) isolés l'un de l'autre.

8. Récipient selon l'une quelconque des revendications 1 à 6, **caractérisé en ce que** la ligne de pliure (7) s'étend du bord supérieur (5) jusqu'à proximité du bord inférieur (6), de sorte à former une zone inférieure de communication fluidique (29) entre les compartiments.

9. Récipient selon l'une quelconque des revendications 1 à 8, **caractérisé en ce qu'**il comprend une deuxième voie d'accès (28) débouchant dans la deuxième chambre de nutrition (16), ladite deuxième voie étant prévue sur un bord transversal du récipient.

10. Récipient selon la revendication 9, **caractérisé en ce que** les première et deuxième voies d'accès (4,28) sont situées sur le bord supérieur (5) du récipient, respectivement de part et d'autre de la ligne de pliure (7).

11. Système comprenant au moins deux éléments reliés entre eux en circuit clos au moyen d'une tubulure, l'un au moins desdits éléments étant un récipient conforme à l'une quelconque des revendications 1 à 10, la tubulure étant connectée par une première extrémité à une voie d'accès du récipient et par une deuxième extrémité à un autre élément du système, de sorte à permettre le passage des cellules et/ou des fluides entre les éléments du système.

12. Procédé de mise en culture de cellules en suspension, comprenant les étapes:
- introduire des cellules en suspension dans un récipient (1) selon la revendication 1 par la première voie d'accès (4),
- plier ledit récipient le long de la ligne de pliure (7),
- incliner le récipient plié dans le plan vertical jusqu'à amener les cellules en suspension dans les chambres de confinement (12,16).

## Patentansprüche

1. Für die Zellkultur vorgesehener Behälter (1), umfassend eine erste und eine zweite Folie (2,3) aus flexiblem, thermoplastischem Material, die in der Nähe ihres Umfangs miteinander verbunden sind, um ein Innenvolumen zu bilden, wobei der Behälter mindestens einen ersten Zugang (4) zum Innenvolumen umfasst, der auf einem transversalen Rand des Behälters liegt und angeordnet ist, um die Einführung und/oder die Gewinnung der Zellen zu ermöglichen, **dadurch gekennzeichnet, dass**:
- der Behälter eine in Längsrichtung verlaufende Faltlinie (7) umfasst, entlang der der Behälter zusammengefaltet werden kann, um auf beiden Seiten der Faltlinie (7) jeweils ein erstes und ein zweites Kompartiment (8,9) in dem Innenvolumen zu bilden,
- das erste Kompartiment (8) mit einem ersten in Längsrichtung verlaufenden Deflektor (10) versehen ist, der angeordnet ist, um zwischen einem in Längsrichtung verlaufenden Rand (11) des Behälters und dem ersten Deflektor (10) eine erste Verschlusskammer (12) und zwischen dem ersten Deflektor (10) und der Faltlinie (7) eine erste Nährkammer (13) zu bilden, wobei die erste Verschluss- und die erste Nährkammer (12,13) fluidisch verbunden sind,
- der erste Zugang (4) in der Nährkammer (12) mündet und
- das zweite Kompartiment (9) mit einem zweiten in Längsrichtung verlaufenden Deflektor (14) versehen ist, der angeordnet ist, um zwischen dem anderen in Längsrichtung verlaufenden Rand (15) des Behälters und dem zweiten Deflektor (14) eine zweite Verschlusskammer (16) und zwischen dem zweiten Deflektor (14) und der Faltlinie (7) eine zweite Nährkammer (17) zu bilden, wobei die zweite Verschluss- und die zweite Nährkammer (16,17) fluidisch verbunden sind.

2. Behälter nach Anspruch 1, **dadurch gekennzeichnet, dass** mindestens ein Deflektor aus einer Schweißnaht (20) zwischen den Folien gebildet ist, wobei sich die Naht in Längsrichtung im Innern des Kompartiments erstreckt und dabei mindestens eine transversale Verbindungszone zwischen der Verschluss- und der Nährkammer bildet.

3. Behälter nach Anspruch 2, **dadurch gekennzeichnet, dass** die Naht von den Rändern des Behälters beabstandet ist, um zwei transversale Verbindungszonen (21, 22), eine obere bzw. eine untere, zu bilden.

4. Behälter nach Anspruch 2 oder 3, **dadurch gekennzeichnet, dass** die Schweißnaht (20) unterbrochen ist.

5. Behälter nach einem der Ansprüche 1 bis 4, **dadurch gekennzeichnet, dass** die Faltlinie (7) mindestens eine Schweißnaht (23) zwischen den Folien umfasst.

6. Behälter nach Anspruch 5, **dadurch gekennzeichnet, dass** die Faltlinie (7) zwei Schweißnähte umfasst, die nebeneinander angeordnet sind.

7. Behälter nach einem der Ansprüche 1 bis 6, **dadurch gekennzeichnet, dass** sich die Faltlinie (7) vom oberen Rand (5) zum unteren Rand (6) des Behälters erstreckt, um zwei Kompartimente (8,9) zu bilden, die voneinander getrennt sind.

8. Behälter nach einem der Ansprüche 1 bis 6, **dadurch gekennzeichnet, dass** sich die Faltlinie (7) vom oberen Rand (5) bis in die Nähe des unteren Rands (6) erstreckt, um eine untere Zone zur fluidischen Verbindung (29) zwischen den Kompartimenten zu bilden.

9. Behälter nach einem der Ansprüche 1 bis 8, **dadurch gekennzeichnet, dass** er einen zweiten Zugang (28) umfasst, der in der zweiten Nährkammer (16) mündet, wobei der zweite Zugang auf einem transversalen Rand des Behälters vorgesehen ist.

10. Behälter nach Anspruch 9, **dadurch gekennzeichnet, dass** der erste und der zweite Zugang (4,28) auf dem oberen Rand (5) des Behälters jeweils beiderseits der Faltlinie (7) liegen.

11. System, umfassend mindestens zwei Elemente, die mithilfe eines Schlauchs in einem geschlossenen Kreislauf miteinander verbunden sind, wobei mindestens eines der Elemente ein Behälter nach einem der Ansprüche 1 bis 10 ist, wobei der Schlauch über ein erstes Ende mit einem Zugang des Behälters und über ein zweites Ende mit einem anderen Element des Systems verbunden ist, um den Durchgang der Zellen und/oder der Fluide zwischen den Elementen des Systems zu ermöglichen .

12. Verfahren zur Kultur von Zellen in Suspension, das die folgenden Schritte umfasst:
- Einführen der suspendierten Zellen in einen Behälter (1) nach Anspruch 1 über den ersten Zugang (4),
- Falten des Behälters entlang der Faltlinie (7),
- Neigen des gefalteten Behälters in vertikaler Ebene, bis die Zellen in Suspension in die Verschlusskammern (12,16) befördert sind.

## Claims

1. A container (1) intended for culturing cells, comprising first and second sheets (2, 3) in a flexible thermoplastic material fixed to each other near their periphery so as to form an inner volume, the container comprising at least one first access channel (4) to the inner volume that is situated on a transverse edge of the container and arranged to allow the introduction and/or recovery of cells, **characterized in that**:
- The container comprises a longitudinal fold line (7) along which the container may be folded so as to form, respectively on either side of said fold line (7), first and second compartments (8, 9) in the inner volume,
- The first compartment (8) is equipped with a first longitudinal baffle (10) that is arranged to form, between a longitudinal edge (11) of the container and said first baffle (10), a first confinement chamber (12) and, between said first baffle (10) and the fold line (7), a first feeding chamber (13), said first confinement and feeding chambers (12, 13) being in fluid communication,
- The first access channel (4) opens into the feeding chamber (12), and
- The second compartment (9) is equipped with a second longitudinal baffle (14) that is arranged to form, between the other longitudinal edge (15) of the container and said second baffle (14), a second confinement chamber (16) and, between said second baffle (14) and the fold line (7), a second feeding chamber (17), said second confinement and feeding chambers (16, 17) being in fluid communication.

2. The container according to claim 1, **characterized in that** at least one baffle is formed by a weld bead (20) between the sheets, said weld bead extending longitudinally inside the compartment by forming at least one cross communication zone between the confinement and feeding chambers.

3. The container according to claim 2, **characterized in that** the bead is spaced from the edges of the container so as to form two cross communication zones (21, 22), respectively upper and lower zones.

4. The container according to claim 2 or 3, **characterized in that** the weld bead (20) is discontinuous.

5. The container according to any one of claims 1 to 4, **characterized in that** the fold line (7) comprises at least one weld bead (23) between the sheets.

6. The container according to claim 5, **characterized in that** the fold line (7) comprises two weld beads arranged one beside the other.

7. The container according to any one of claims 1 to 6, **characterized in that** the fold line (7) extends from the upper edge (5) to the lower edge (6) of the container, so as to form two compartments (8, 9) isolated from one another.

8. The container according to any one of claims 1 to 6, **characterized in that** the fold line (7) extends from the upper edge (5) to near the lower edge (6), so as to form a lower fluid communication (29) zone between the compartments.

9. The container according to any one of claims 1 to 8, **characterized in that** the container comprises a second access channel (28) opening into the second feeding chamber (16), said second channel being provided on a transverse edge of the container.

10. The container according to claim 9, **characterized in that** the first and second access channels (4, 28) are situated on the upper edge (5) of the container, respectively on either side of the fold line (7).

11. A system comprising at least two elements interconnected in closed circuit by means of tubing, at least one of said elements being a container in conformance with any one of claims 1 to 10, the tubing being connected by a first end to an access channel of the container and by a second end to another element of the system, so as to enable the passage of cells and/or fluids between the elements of the system.

12. A method of culturing cells in suspension, comprising the steps of:
- Introducing cells in suspension into a container (1) according to claim 1 by the first access channel (4),
- Folding said container along the fold line (7),
- Tilting the folded container in the vertical plane until the suspended cells move into the confinement chambers (12, 16).
